# EUROPEAN PATENT APPLICATION

(11) **EP 4 555 917 A1**
(43) Date of publication of application: **21.05.2025**
(21) Application number: 23210416.6
(22) Date of filing: 16.11.2023
(51) Int. Cl.: A61B 3/032, A61B 3/036

(54) **METHOD AND APPARATUS FOR DETERMINING AT LEAST ONE REFRACTIVE VALUE OF AN EYE**

(71) Applicant: Carl Zeiss Vision International GmbH, 73430 Aalen (DE)
(72) Inventor: Buehren, Tobias, 73447 Oberkochen (DE); Avenhaus, Malte, 73447 Oberkochen (DE); Wiedemann, Doreen, 73447 Oberkochen (DE); Walch, Marcel, 73447 Oberkochen (DE)
(74) Representative: Altmann Stößel Dick Patentanwälte PartG mbB

(57) **Abstract**

The present invention refers to a method (218), a computer program, and an apparatus (110) for determining at least one refractive value (216) of an eye (112) of a person (114) and to a related method (210) for producing at least one ophthalmic lens (212) for the eye (112) of the person (114). The method (218) comprises the following steps:
a) (220) displaying a visual stimulus (120) to an eye (112) of a person (114), wherein the visual stimulus (120) comprises a plurality of concentric radial lines (126, 126,', ...), wherein each radial line (126, 126,', ...) has a different angular value (128, 128,', ...) with respect to a center (130) of the visual stimulus (120);
b) (224) recording a reaction by the person (114) to the visual stimulus (120); and
c) (226) determining at least one refractive value (216) of the eye (112) of the person (114) by evaluating the reaction by the person (114) to the visual stimulus (120),
wherein step a) (220) comprises that each radial line (126, 126,', ...) is projected at a particular defocus plane to the eye (112) of the person (114), whereby a particular dioptric power is provided to the eye (112) of the person (114), and wherein step b) (224) comprises that recording the reaction by the person (114) to the visual stimulus (120) indicates that a particular radial line (144) at a particular angular value (146) has an appearance of maximum sharpness (148) to the eye (112) of the person (114).

## Description

### Field of the invention

The present invention refers to a method, a computer program, and an apparatus for determining at least one refractive value of an eye of a person and to a related method for producing at least one ophthalmic lens for an eye of a person.

### Related art

A use of rotating dials for subjective sight-tests in order to determine best sphere, cylinder, astigmatic axis, and power dates back to the nineteenth century. As described in Keirl A.W. and Christie C., Clinical Optics & Refraction, 1st Edition, Butterworth-Heinemann, Oxford, 2007, pp. 117-123, and Elliot D.B., Clinical Procedures in Primary Eye Care, Butterworth-Heinemann, Oxford, 2003, pp. 117-123, Lancaster credited Donders for using a star-shaped figure, which comprises a plurality of concentric radial lines as a practical test for astigmatism as early as 1860. Green perfected the astigmatic dial while working with Donders and Snellen from 1866 to 1869. In 1899, Verhoeff suggested a chart having two principal meridians to measure an amount of astigmatism. Variations of a so-denoted "cross-cylinder method" became available, including sunburst charts or clock dials having lines at intervals of 30° or at more frequent intervals, most popularly at 10°.

Keirl A.W. and Christie C., see above, further describe a so-denoted "fan-and-block-technique", which is frequently implemented in sight-test units to determine the best sphere, the axis and magnitude of astigmatism. Herein, a fan is used to determine a presence of astigmatism and its principal axes, while blocks are used in conjunction with cylindrical lenses in order to neutralize the astigmatism. In particular, the fan-and-block-technique may comprise the following two elements:
- a fan chart comprising a plurality of concentric radial lines at 10° intervals, each subtending an equivalent of 6/18 limb at a distance of 6 m, arranged around a central rotation disc marked with an arrowhead known as "Maddox V", wherein numbers around the radial lines indicate the axis for a correcting negative cylinder; and
- a central rotatable disc comprising
   ∘ the Maddox V arrowhead; and
   ∘ two blocks of parallel lines set at 90° to each other,
wherein the central rotatable disc is designed in a manner that, when the Maddox V arrowhead is rotated towards the line of the fan having a maximum sharpness to the eye of the person, the blocks align with the principal meridians of the eye of the person.

A so-denoted "Test Chart 2020" by Thomson Software Solutions, which is available under https://www.thomson-software-solutions.com/test-chart-xpert-3di/ comprises a plurality of test charts and vision tests, binocular vision tests and refraction stimuli. For their use, a software has to be installed on personal computer, a monitor mounted on a wall, a screen viewing distance entered, and a calibration test performed. Included is a fan and block test for identifying and correcting astigmatism.

Andrew Carkeet, Jia Hao Ng, and Jia Sheng Choo, Bearing fixing: A new computer algorithm method for subjective determination of astigmatism, Ophthalmic and Physiological Optics 41 (5), 2021, present the principles of bearing fixing, a computercontrolled procedure for subjective determination of astigmatism, and compare it with conventional clinician-controlled Jackson crossed-cylinder refraction. Astigmatism was measured using bearing fixing and Jackson cross-cylinder twice on 20 visually normal participants aged between 18 and 34 years. After final sphere adjustment, acuity measurements were made with each refraction estimate. Bearing fixing results could be obtained for all participants. Mean bearing fixing cylinder magnitude was slightly higher than Jackson cross-cylinder measures of cylinder magnitude, by 0.05 D. Using vector analysis to take into account cylinder power and axis, mean bearing fixing astigmatism was not significantly different from cross-cylinder astigmatism, but did have higher test-retest variability (p < 0.05). Acuity with bearing fixing and cross-cylinder corrections did not significantly differ in average value or repeatability.

Further multiple alternative approaches to display test patterns are known, in particular virtual refraction systems (e.g. Vision Optimizer a System with refraction elements above the head of the patient), digital infinite refraction (e.g. Vision-S 700 Essilor), or guided refraction systems (e.g. Chronos Topcon or VASRTM Vision Inc). In addition, in-store refraction units or table-top concepts, mobile phone subjective refraction approaches such as used in conjunction with head mounted VR-goggles (e.g. EyeNetra) or smartphone apps including a desktop monitor (e.g., Opternative) provide state-of-the art solutions. Further, CREAL SA, 1024 Ecublens, Switzerland, presents under the title "Whitepaper: CREAL'S digital light field", which is available under https://creal.com/technology/ a light-field display configured for displaying virtual images in desired focal distances.

EP 3 730 038 A1 discloses a system and method for interactively measuring ocular refractive errors, addition and power of reading glasses without need of an optical component that would change optical vergence of a target. The system can measure the distance from a user's eyes to either or both border of interval of clear vision, for one orientation or two perpendicular orientations. The measurements together with the user's age can be used to estimate sphero-cylindrical refraction, addition and power of reading glasses. The system can use different sizes, directions and colors of targets which can change with said distance or user interaction.

US 2020/0397279 A1 discloses methods, systems, and devices for measuring eye refraction with a mobile device application without a lens. An exemplary method includes measuring a distance between a patient and a mobile device, presenting to the patient one or more visual targets sized and shaped to represent perfect vision such as by using Vernier targets or grating targets, instructing the patient to indicate whether the patient can accurately read the visual targets and, if not, to move closer to the mobile device until the patient can accurately read the visual targets, calculating a vision prescription for the patient based on the visual targets and a final distance between the patient and the mobile device, and displaying the vision prescription for the patient.

### Problem to be solved

In particular with respect to the disclosure of the so-denoted "fan-and-block-technique", as exemplarily described by Keirl A.W. et al., see above, it is therefore an objective of the present invention to provide a method, a computer program, and an apparatus for determining at least one refractive value of an eye of a person and a related method for producing at least one ophthalmic lens for an eye of a person, which at least partially overcome the above-mentioned problems of the state of the art.

It is a particular objective of the present invention to be able to determine at least one refractive value of an eye of a person, especially with regard to a spherical power, an cylindrical power and an cylindrical axis, by applying a reliable, easy and intuitive approach. Thereby, it is desirable to determine the at least one refractive value without requiring an ophthalmologist, optician or optometrist, a set of measuring glasses or a sophisticated device, such as an autorefractive device, designated for this purpose. In particular, it is desirable to determine the at least one refractive value in a fashion which is applicable on a global scale to all kinds of persons, whereby difficulties with communication or compliance can be avoided as far as possible.

### Summary of the invention

This problem is solved by a method, a computer program, and an apparatus for determining at least one refractive value of an eye of a person and a related method for producing at least one ophthalmic lens for an eye of a person having the features of the independent claims. Preferred embodiments, which might be implemented in an isolated fashion or in any arbitrary combination, are listed in the dependent claims or throughout the following description.

In a first aspect, the present invention relates to a method for determining at least one refractive value of an eye of a person. The method comprises the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps not listed herein.

The method for determining at least one refractive value of an eye of a person comprises the following steps:
a) displaying a visual stimulus to an eye of a person, wherein the visual stimulus comprises a plurality of concentric radial lines, wherein each radial line has a different angular value with respect to a center of the visual stimulus, wherein each radial line is projected at a particular defocus plane to the eye of the person, whereby a particular dioptric power is provided to the eye of the person;
b) recording a reaction by the person to the visual stimulus indicating that a particular radial line at a particular angular value has an appearance of maximum sharpness to the eye of the person; and
c) determining at least one refractive value of the eye of the person by evaluating the reaction by the person to the visual stimulus.

The present method is configured for determining at least one refractive value of an eye of a person. Instead of the term "person", a different term, such as "subject", "test person", "user", or "wearer", may also be applicable. The person may perform the method autonomously; however, she may be accompanied by a further person supporting the person, wherein the further person may, preferably, selected from at least one of a parent, a nurse, an optician, an apprentice, an ophthalmologist, a optics sales person, a technical photographer. However, a further person which may accompany the person may also be conceivable.

As generally used, the term "determining", or any grammatical variation thereof refers to a process configured for generating at least one representative result. The representative result may be determined in a process, wherein at least one step of the process may be selected from at least one of a measurement step; an evaluation step; or a displaying step. For determining the representative result, at least one measurement may be performed. Data generated in this measurement may be evaluated. The representative result may be data retrieved in the evaluation process. The representative result may be displayed. With particular regard to the present invention, the representative result comprises outcome data comprising at least one refractive value of the eye of the person. The outcome data may be displayed on at least one monitor.

As further generally used, the terms "refraction" or "refractive" refer to a bending of incident light entering the interior of the eye of the person via a pupil in a manner that the incident light may, in particular owing to a form of the eye, not be focused appropriately on the retina of the eye, resulting particularly in a blurred vision of the person. Herein, the term "refractive value" is a number indicating a strength of the bending of incident light. In particular, the present method is configured for determining at least one astigmatic effect of an eye of a person, wherein the term "astigmatic effect" refers to a condition in which an optical system comprising one eye of the person forms two individual line images of a point object, typically, as a result of a toricity of at least one refracting surface of the eye of the person. As a result thereof, the refraction causes that light which impinges on an eye is distributed on the retina in an unequal fashion, which may, in particular, result in a distorted and/or blurred vision of the person. The method may be implemented for being performed for exactly one eye of the person; it may be repeated for a further eye of the person, wherein the other eye of the person can be covered, particularly initiated by a corresponding graphical user interface, which may be configured for assisting the person in this respect.

As used herein, the at least one refractive value of the eye of the person comprises values of:
- a spherical power, abbreviated to "Sph";
- a cylindrical power, abbreviated to "Cyl"; and
- a cylinder axis, abbreviated to "A".

Based on standard ISO 13666:2019 (referred to as "Standard" in the following), Section 3.12.2, the term "spherical power" refers to a value of a back vertex power of an ophthalmic lens, including the lens of the eye of the person, or for a back vertex power in one of two meridians of an astigmatic-power lens, depending on a principal meridian chosen for reference. Based on the Standard, Section 3.13.2, the term "principal meridian" is one of two mutually perpendicular meridians of the astigmatic-power lens that are parallel to the two line foci. Herein, the first principal meridian algebraically has the lower vertex power, while the second principal meridian algebraically has the higher vertex power, according to the Standard, Section 3.13.3-4. Based on the Standard, Sections 3.13.6-7, the term "cylindrical power" refers to an algebraic difference between the power of the principal meridian chosen for reference subtracted from the power of the other perpendicular meridian. Based on the Standard, Section 3.13.8, the term "cylinder axis" refers to a direction of the principal meridian of a lens whose vertex power is chosen for reference and, therefore, corresponds to the meridian direction of the astigmatism of the eye of the person. Based on the Standard, Sections 3.10.2-3, the term "dioptric power" comprises both the spherical power and the cylindrical power of the ophthalmic lens, if a prismatic effects are neglected.

For a purpose of exerting an influence on the at least one refractive value of an eye of a person, an ophthalmic lens may be used. Based on the Standard, Section 3.5.1, the term "ophthalmic lens" refers to an optical lens which is configured for a purpose of at least one of a measurement, a correction or a protection of the eye. According to the Standard, Section 3.5.2, the term "spectacle lens" relates to a type of ophthalmic lens, which is worn in front of, but not in contact with, the eyeball, whereas the term "contact lens" relates to a further type of ophthalmic lens, which is worn in in contact with the eyeball. However, further types of ophthalmic lenses are feasible.

In a particularly preferred embodiment, the method for determining at least one refractive value of an eye of a person may be a computer-implemented method. As generally used, the term "computer-implemented method" refers to a method, which involves at least one device, specifically a computer, particularly connected to a computer network. A plurality of devices may be connected, particularly for transmitting data, via a network by using at least one connection interface at any one of the devices of the plurality of devices. The computer-implemented method may be implemented as at least one computer program that may be provided on a storage medium carrying the computer program, whereby at least one step of the method may be performed by using the at least one computer program. Preferably any step of the method is performed using the at least one computer program. Alternatively, the at least one computer program may be accessible by an apparatus which may be adapted for performing the method via a network, in particular via an in-house network, via internet, or via a cloud.

According to step a), a visual stimulus is displayed to an eye of a person. As generally used, the term "visual stimulus" refers to a graphical presentation of an item, particularly an item that is known or reasonably to be expected by the person skilled in the art to be considerably suitable for determining at least one refractive value of an eye of a person. Such an item can also be denoted by the term "optotype". The visual stimulus may particularly be suitable if it is perceptible by the person, especially due to a contrast between the visual stimulus and a background that allows the eye of the person to distinguish between the visual stimulus and the background.

As indicated above, the visual stimulus comprises a plurality of concentric radial lines. As generally used, the term "line" refers to a straight two-dimensional curve having a length which exceeds a lateral extension of the line, preferably by a factor of at least 10, more preferred of at least 25, especially of at least 50. Herein, the line may, preferably, be an continuous elongated line; however, using a dashed line may also be feasible. Further, the line may be black or may exhibit at least one color. Further, the line may exhibit a uniform lateral extension, or the lateral extension may vary along the length of the line. Further, the line may, preferably, exhibit a uniform contrast; however, the contrast may slightly vary along the length of the line. Further embodiments of the line may also be feasible.

As further generally used, the term "radial" refers to a direction originating at a central point and departing from the central point in a straight manner. In general, the central point may be a "center" in a sense that a particular line may originate at the central point. However, with regard to the present invention, the central point functions as a "center" in a sense that a particular line may not originate at the central point but in a particular distance from the central point. As further generally used, the term "concentric" relates to an arrangement of a plurality of radial lines, wherein each radial line is oriented with respect to an identical central point. Herein, the plurality of the radial lines may, preferably, originate at the same particular distance from the central point; however, using different distances from the central point for some or all radial lines may also be feasible.

As further indicated above, each concentric radial line as comprised by the visual stimulus has a different angular value with respect to the center of the visual stimulus. As generally used, the term "angular value" refers to a number indicating a relative direction to a predetermined radial direction. In a particularly preferred embodiment, the concentric radial lines may be arranged as a fan having a starburst pattern around the center of the visual stimulus. Herein, the terms "fan" is used in a sense as disclosed by Keirl A.W. et al., see above, in connection with the so-denoted "fan-and-block-technique", which results in a so-denoted "starburst pattern" which appears to originate from the center of the visual stimulus.

Preferably, the visual stimulus may be a monochrome starburst pattern; however, using a chromatic starburst pattern may also be feasible.

In a further particularly preferred embodiment, the concentric radial lines may be arranged in equidistant steps; however, using different angular distances between adjacent lines for some or for all concentric radial lines may also be feasible. In a preferred embodiment, the visual stimulus may comprise 6, 8, 9, 12, 15, 18, 24, 30, 36, 48, 60, 72, 90, 96, or 120 of concentric radial lines, thus, resulting in an angular distance of 3°, 3.75°, 4°, 5°, 6°, 7.5°, 10°, 12°, 15°, 20°, 24°, 30°, 40°, 45°, or 60°, respectively. However, using a different number of concentric radial lines may also be feasible.

In a particularly preferred embodiment, a projection device configured for displaying the visual stimulus to the eye of the person according to step a) may be used. As used herein, the term "projection device" refers to an electronic visual display device designated for a presentation of the visual stimulus, wherein the electronic visual display device is configured for projecting each radial line of the visual stimulus at a particular defocus plane to the eye of the person. As particularly preferred, the projection device may be located in front of the person in a manner that it may face an eye of the person. However, using at least one additional optical element, especially at least one of an optical mirror or a beam splitter, may also be conceivable. In a preferred embodiment, at least one holding element may, additionally, be attached to the projection device, wherein the at least one holding element may be configured for maintaining a position of the projection device with respect to an eye of the person.

In general, the projection device may, preferably, be comprised by at least one of
- a monitor comprised by a desktop computer;
- a touchscreen comprised by a mobile communication device.

However, further types of screens may also be feasible. As generally used, the term "desktop computer" refers to a computer in a housing suitable for use as a workstation computer on top of a desk but, sometimes, also placed below a desk. As further generally used, the term "mobile communication device" refers to an electronic device configured for communicating with at least one further electronic device, which can be carried by the person and, may thus, move together with the person. In general, the mobile communication device comprises at least one of a screen and a processing device and, optionally, a camera, wherein a mobile operating system running on the processing unit may be configured for facilitating a use of software, internet, and multimedia functionalities, in particular by using a wireless communications protocol, such as Wi-Fi or Bluetooth. Further, the processing device may comprise an evaluation device as described below in more detail. Further, the mobile communication device may comprise at least one sensor which may, in particular, be selected from at least one of a motion sensor, a gyro sensor, an accelerometer, a proximity sensor, a magnetometer, or a barometer; however, using a further kind of sensor may also be feasible. Further, the mobile communication device may comprise at least one actuator, in particular a vibration motor. Further, the mobile communication device may comprise at least one microphone configured for recording at least one of an acoustic response by the person.

In particular, the mobile communication device may be selected from at least one of:
- a smartphone;
- a smart watch;
- a smart glass;
- an augmented reality glass;
- a virtual reality glass;
- a laptop;
- a tablet;
- a smart television.

As generally used, the term "smartphone" refers to a mobile phone having computer functionalities and connectivity and may, additionally, comprise at least one of a camera, a sensor or an actuator. As generally used, the term "smart watch" refers to an electronic wristwatch which has computer functionalities and connectivity and may comprise at least one of a sensor or an actuator. As generally used, the term "smart glass" refers to a wearable spectacle lens having computer functionalities and connectivity, wherein, in addition to the visual reception of the wearer through the ophthalmic lens, further information may be provided. As generally used, the term "augmented reality glass" refers to a controllable ophthalmic lens being configured for providing access to augmented reality for a wearer. As generally used, the term "virtual reality headset" refers to a head-mounted display designed for providing access to virtual reality for a wearer. As generally used, the term "laptop" refers to a particular type of computer having a screen being movably attached to a housing, wherein the screen can be folded onto the housing. As generally used, the term "tablet" refers to a portable, flat touch-screen computer. As further generally used, the term "smart television" refers to a television set which, further, comprises computer functionalities and connectivity.

In accordance with the present invention, each radial line is projected at a particular defocus plane to the eye of the person. Preferably each of at least two radial lines, more preferred each radial line, is projected at a different defocus plane to the eye of the person; however, at least two radial lines may be projected at the same defocus plane to the eye of the person as long as sufficient radial lines for the purposes of the present invention may be projected at different defocus planes to the eye of the person. As generally used, the terms "focal plane" or "focus plane" refer to an imaginary plane, which is perpendicular to an optic axis and passes through a focal point. As further generally used, the term "defocus plane" relates to a further imaginary plane, which is spatially displaced with respect to a focus plane in a parallel manner. Accordingly, each radial line as projected at a different defocus plane is recognized by the eye of the person as a virtual three-dimensional image having a different distance to the eye of the person. Displaying the radial lines in this manner corresponds to generating a different dioptric effect by each radial line to the eye of the person. As a consequence, each radial line provides a particular dioptric power to the eye of the person.

In a particularly preferred embodiment, it is proposed to set a difference between the defocus planes for adjacent radial lines by using a dioptric step. As a result thereof, two adjacent radial lines exhibit a difference in distance as recognized by the eye of the person, which corresponds to a dioptric step. As generally used, the term "dioptric step" relates to a fixed difference between two dioptric powers as expressed in diopters. In a particular embodiment, the dioptric step may be selected from a value of 0.125 D, 0.25 D, 0.5 D, 0.75 D, or 1.0 D; however, using a different value for the dioptric step may also be feasible.

In order to project each radial line at the particular defocus plane to the eye of the person, the projection device may, preferably, be configured for using a virtual display technique, wherein the projection device may, particularly, be selected from at least one of:
- a light-field display ;
- an holographic optical element;
- a retinal laser beam projection.

However, using a different type of projection device may also be conceivable. As generally used, the term "light field" refers to vector function indicating an amount of light propagating in a particular direction at a particular spatial point. Abased hereon, a "light-field display" is designed for generating a rendered 3D model or an imaged scene that as obtained from a plurality of viewpoints, in particular by using a moving camera or an array of cameras. As further generally used, the term "holographic optical element" relates to an optical element configured for generating at least one holographic image or "hologram" by using optical diffraction. Herein, the term "optical diffraction" refers to a propagation of light in presence of an obstacle denoted as "optical diffraction element", such as a slit or a diffraction grating. For a purpose of generating a volume hologram, a known "holographic display" can be used. As further generally used, the term "retinal laser beam projection" relates to a device which is configured for projecting a laser beam onto the retina of the eye of the person. As a result, the person directly recognizes a visual stimulus which is generated at the retina of the eye by using a laser beam.

According to step b), a reaction by the person to the visual stimulus is recorded. As generally used, the term "reaction" refers to an action of the person in response to a visual stimulus displayed to an eye of the person. As further generally used, the term "recording" or any grammatical variation thereof relates to a process of recognizing a reaction of the person by observing a behavior of the person, which may be expressed by a measurable signal, in particular an electronic signal or an optical signal, which may be provided by at least one input element as described below in more detail.

In a particularly preferred embodiment, at least one input element configured for recording a reaction by the person to the visual stimulus according to step b) may be used. As used herein, the term "input element" refers to an element which is configured for monitoring an occurrence of an event by providing or interrupting a monitoring signal when the event occurs. In particular, the at least one input element may be configured for recording at least one of:
- a tactile response by the person;
- an acoustic response by the person;
- a gesture of the person.

Herein, recording the reaction by the person to the visual stimulus can be initiated by using a graphical user interface, which may be configured for assisting the person in this respect, as described below in more detail.

As generally used, the term "tactile response" refers to a manual touching of a mechanical or a virtual element by the person. For a purpose of recording the at least one tactile response by the person, at least one of a mechanical button, or a virtual button can, particularly, be used, wherein the virtual button may be displayed on the touchscreen, preferably placed on the projection device close to a rotation element, preferably a mechanical rotation element or a virtual rotation element, as described below in more detail, but not overlapping it. As an alternative, a combined rotation and input element, which may comprise the function of both the rotation element and the input element, can be used in this fashion. By way of example, touching the combined rotation and input element in a particular fashion, especially by pressing or stopping it or a portion thereof, may be considered as recording the desired reaction by the person to the visual stimulus. Alternatively, or in addition, recording that the combined rotation and input element has been stopped for a predefined time interval can be interpreted as indicating the desired reaction by the person to the visual stimulus. However, different types of input elements configured for recording the at least one tactile response may also be conceivable.

As further generally used, the term "acoustic response" refers to receiving a sound generated by the person, wherein the sound is interpreted as indicating the desired reaction by the person to the visual stimulus. For a purpose of recording the at least one acoustic response by the person, at least one microphone can be used; however, using a different type of element may also be feasible. As further generally used, the term "gesture" refers to a particular behavior of the person which can be interpreted as the desired reaction by the person to the visual stimulus. For a purpose of recording the at least one gesture of the person, at least one monitoring device, especially selected from a camera, a motion sensor, or an eye tracker, can be used. In particular, at least one front camera placed in the vicinity of the projection device, especially above the projection device, may be preferred; however, using a different arrangement and type of the monitoring device may also be conceivable.

Further according to step b), the reaction by the person to the visual stimulus indicates that a particular radial line at a particular angular value has an appearance of maximum sharpness to the eye of the person. As generally used, the term "indicating" or any grammatical variation thereof relates to a process of providing a piece of information that an event is occurring or has just occurred. As further generally used, the term "appearance" refers to a process of recognizing an event by a person. As further generally used, the term "sharpness" relates to a contrast between an object and an area surrounding the object at least partially. As further generally used, the term "maximum" refers to an extreme value indicating a highest value in a set of values. As still further generally used, the term "minimum" refers to a further extreme value indicating a lowest value in a set of values.

With particular regard to the present invention, the reaction by the person to the visual stimulus provides the piece of information that the person recognizes that a contrast in a particular radial line, which is displayed to the eye of the person at a particular angular value, shows a contrast which is higher than or as high as the other radial lines comprised by the visual stimulus. Since each radial line is projected according to step a) at a particular defocus plane to the eye of the person, whereby a particular dioptric power is provided to the eye of the person, the reaction by the person to the visual stimulus provides the piece of information that the eye of the person can see sharp the particular radial line, which is projected at the particular defocus plane.

According to step c), the at least one refractive value of an eye of the person is determined by evaluating the reaction by the person to the visual stimulus. As generally used, the term "determining" or any grammatical variation thereof refers to a process of generating at least one representative result, such as a plurality of representative results. For a definition of the expression "refractive value", reference can be made to the definition above. As further used herein, the term "evaluating" or any grammatical variation thereof refers to applying at least one algorithm to extract at least one piece of information from the at least one reaction by the person to the visual stimulus. The at least one algorithm may be configured for determining the at least one refractive value of an eye of the person by evaluating a relationship between the angular value of a particular radial line at a particular angular value and the reaction by the person to the appearance of the maximum sharpness to the eye of the person of the particular radial line according to a scheme. As indicated above, the piece of information that the eye of the person can recognize the particular radial line being projected at the particular defocus plane as sharp is used for determining the at least one refractive value by applying at least one appropriate algorithm.

In a particularly preferred embodiment, the method according to the present invention may, further, comprise the following additional step:
d) rotating the visual stimulus by the person, wherein the angular value of each radial line with respect to the center of the visual stimulus is altered, whereby a rotated visual stimulus is obtained.

Herein, recording the reaction by the person to the visual stimulus according to step b) may, particularly, comprise indicating that a particular radial line at a particular angular value in the rotated visual stimulus has the appearance of maximum sharpness to the eye of the person and wherein determining the at least one refractive value of the eye of the person according to step c) further comprises evaluating the reaction by the person to the rotated visual stimulus.

According to step d), the visual stimulus is rotated by the person, whereby a rotated visual stimulus is obtained. As generally used herein, the term "rotating" or any grammatical variation thereof refers to a process of moving an object in a manner that a center of the object is maintained, while an angular value of the object is, concurrently, altered. With regard to the present invention, rotating the visual stimulus comprises maintaining the center of the visual stimulus and, concurrently, altering the angular value of each radial line. As particularly preferred, the projection device as described above or below in more detail, may further be configured for displaying the rotation of the at least one visual stimulus by the at least one angular value. As consequence of rotating the visual stimulus in this manner, each radial line which provides a particular dioptric power to the eye of the person is moved around the center of the visual stimulus.

In a particularly preferred embodiment, at least one rotation element configured for rotating the visual stimulus by the person by the at least one angular value according to step d) may be used. As used herein, the term "rotation element" refers to an element which is configured for rotating the visual stimulus by the person to obtain the rotated visual stimulus in a manner that the center of the visual stimulus maintained, while the angular value of each radial line with respect to the center of the visual stimulus is, concurrently, altered. Preferably, the at least one rotation element may be selected from at least one of a mechanical rotation element or a virtual rotation element.

As generally used, the term "mechanical rotation element" relates to an element, which can be manually touched by the person to induce a rotation of an object, wherein at least one of an intensity, a duration, or a direction of a touching the element by the person may be configured for inducing at least one of a velocity or a direction of the rotation of the object. Further, removing a hand of the person from the mechanical rotation element may induce a stop of the rotation of the object. In particular, the mechanical rotation element may be selected from at least one of a turning knob, a button, or a switch. However, using a different kind of mechanical element may also be feasible. Especially, the mechanical rotation element may be placed in a vicinity of the projection device in order to maintain the concentration of the person towards the visual stimulus, while the person is rotating the visual stimulus by actively executing a mechanical action.

As further generally used, the term "virtual rotation element" relates to a virtual element displayed on a screen, especially on the same projection device which is used for displaying the at least one visual stimulus. In a preferred embodiment, the screen may be a touchscreen, wherein the virtual element can be manually touched by the person on the touchscreen to induce a rotation of an object, wherein at least one of an intensity, a duration, or a direction of a touching the virtual element by the person may be configured for inducing at least one of a velocity or a direction of the rotation of the object. Further, removing a hand of the person from the virtual rotation element may induce a stop of the rotation of the object. Especially, the virtual rotation element may be placed on the projection device close to the visual stimulus, but not overlapping it, in order to maintain the concentration of the person towards the visual stimulus, while the person is virtually rotating the visual stimulus on the projection device. In particular, the virtual rotation element may be selected from at least one of a virtual turning knob, a virtual button, or a virtual switch, which may be displayed on the screen to resemble to the person a mechanical equivalent selected from a turning knob, a button, or a switch. However, using a different kind of virtual element may also be feasible. Alternatively, or in addition, the virtual rotation element may refer to an arbitrary element which may be configured for voice control by receiving an acoustic response by the person, wherein the acoustic response may be configured for inducing or stopping a rotation of the object and for adjusting a velocity or a direction of the rotation of the object. However, still further alternative embodiments of the virtual rotation element may be feasible.

With particular regard to the present invention, the reaction by the person to the visual stimulus provides the piece of information that the person recognizes that a contrast in a particular radial line, which is displayed to the eye of the person at a particular angular value, shows a contrast which is higher than or as high as the other radial lines comprised by the visual stimulus. Since each radial line is projected according to step a) at a particular defocus plane to the eye of the person, whereby a particular dioptric power is provided to the eye of the person, and since the visual stimulus is rotated by the person according to step d) to obtain the visual stimulus, the reaction by the person to the visual stimulus provides the piece of information that the eye of the person can see sharp the particular radial line, which is projected at the particular defocus plane.

In a particularly preferred embodiment, the at least one refractive value of the eye of the person may comprise at least one value of at least one refraction ellipse, preferably all following values of the at least one refraction ellipse:
- a first spatial direction, which indicates
   ∘ a maximum value, or
   ∘ a minimum value
   of the dioptric power,
- a second spatial direction, which indicates
   ∘ a minimum value of the dioptric power, if the first spatial direction indicates the maximum value of the dioptric power, or
   ∘ a maximum value of the dioptric power if the first spatial direction indicates the minimum value of the dioptric power; and
- an angular value, which indicates a direction of
   ∘ the maximum value, or
   ∘ the minimum value
   of the dioptric power.

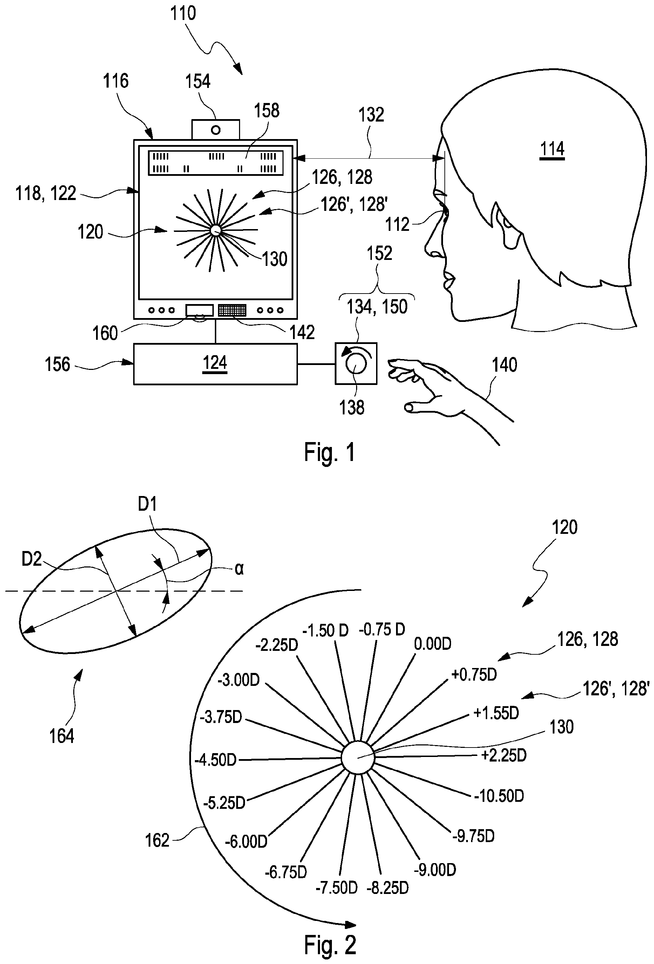

Herein, the term "dioptric power" is used as defined above. Further information about the refraction ellipse can be found below in Figure 2 and the corresponding description.

In this particularly preferred embodiment, step d) may comprise determining concurrently
- a principal meridian from the particular angular value at which the particular radial line has the appearance of maximum sharpness to the eye of the person; and
- a difference between the maximum value and the minimum value of the dioptric power from the particular dioptric value at which the particular radial line is projected at the particular defocus plane.

For a definition of the term "principal meridian", reference can be made to the definition as provided above.

In a particularly preferred embodiment, the method according to the present invention may, further, comprise the following additional steps:
e) further rotating the visual stimulus by the person, whereby a further rotated visual stimulus is obtained, wherein the angular value of each radial line with respect to the center of the visual stimulus is further altered;
f) recording a further reaction by the person to the further rotated visual stimulus indicating that a further particular radial line at a further particular angular value has the appearance of maximum sharpness to the eye of the person, wherein the further particular angular value is perpendicular to the particular angular value according to step d); and
g) determining at least one further refractive value of the eye of the person by evaluating the further reaction by the person to the further rotated visual stimulus.

As used herein, the term "further" indicates that at least one process is repeated at least once, preferably once. In particular, by further rotating the visual stimulus by the person according to step e), a further rotated visual stimulus in addition to step d) is obtained, wherein the angular value of each radial line with respect to the center of the visual stimulus is, compared to step d), additionally altered. Similarly, a further reaction by the person to the further rotated visual stimulus is recorded according to step f) in a manner that it indicates that a further particular radial line at a further particular angular value also has the appearance of maximum sharpness to the eye of the person. In a particular preferred embodiment, the further particular angular value is selected to be perpendicular to the particular angular value as recorded at step d). Finally, the at least one further refractive value of the eye of the person is determined according to step g) by further evaluating both the reaction by the person to the rotated visual stimulus as recorded at step d) and the further reaction by the person to the further rotated visual stimulus as recorded at step f).

In a particularly preferred embodiment, step g) may, preferably, comprise determining
- a perpendicular meridian being perpendicular to the principal meridian from the further particular angular value at which the further particular radial line has the appearance of maximum sharpness to the eye of the person;
- the maximum value of the dioptric power from a further particular dioptric value at which the further particular radial line is projected at the further particular defocus plane; and
- the minimum value of the dioptric power by using the maximum value of the dioptric power and the difference between the maximum value and the minimum value of the dioptric power.

Further information about can be found below in the Figures and in the corresponding description.

Determining the at least one refractive value of an eye of the person may be performed in accordance with a predefined scheme, however, artificial intelligence, in particular machine learning, may also be applied, especially by using a neuronal network. As generally used, the term "machine learning" refers to a process using artificial intelligence to automatically generate a statistical model for at least one of classification or regression. A machine learning algorithm configured for generating the desired model based on a large number of training data sets can, preferably, be used. Herein, the machine learning algorithm can be a supervised algorithm or a self-learning algorithm. The machine learning algorithm can use or comprise a neural network, preferably, developed into a trained neural network by using the at least one training data set. The neural network may comprise at least one element selected from hierarchical decision trees, Hough forest, regression forest, Convolutional Neural Network (CNN), Deep Neural Network (DNN) Residual Neural Network, Pixel-wise Voting, Pixel-wise Fusion Network, Deep learning. Alternatively, or additionally, using at least one other artificial intelligence method, such as a kernel method, especially a Support Vector Machine (SVM), may also be conceivable.

In a particularly preferred embodiment, at least one evaluation device configured for determining at least one refractive value of the eye of the person by evaluating the reaction by the person to the visual stimulus according to step b) may be used. As further generally used, the term "evaluation device" refers to a processing device which is configured for executing the at least one algorithm as described above, wherein the processing of the at least one algorithm may be performed in at least one of a consecutive or a parallel manner. In a preferred embodiment, all method steps can be performed by using a single processing device, such as a computer, especially a desktop computer or a mobile communication device, as disclosed above and below in more detail. Herein, the single processing device may be configured for exclusively performing at least one computer program, in particular at least one line of computer program code configured for executing at least one algorithm, as used in at least one of the methods according to the present invention. For this purpose, the computer program as executed on the single processing device may comprise all instructions causing the computer to carry out the at least one of the methods according to the present invention. Alternatively or in addition, at least one method step can be performed by using at least one remote processing device, especially selected from at least one of a server or a cloud server, which may, preferably, not be located at the site of the person when executing the at least one method step. In this further embodiment, the computer program may comprise at least one remote portion to be executed by the at least one remote processing device to carry out the at least one method step.

Further, the evaluation device may comprise at least one communication interface configured for at least one of forwarding or receiving data to or from the at least one remote portion of the computer program. As generally used, the term "communication interface" refers a transmission channel configured for a transmission of data. Preferably, the communication interface may be arranged as a unidirectional interface configured for forwarding at least one piece of data into a single direction, from at least one input interface to the processing device, or from the evaluation device to at least one output interface. Alternatively, the communication interface may be arranged as a bidirectional interface configured for forwarding at least one piece of data into one of two directions, from the evaluation comprising to both an input interface and an output interface, or vice versa.

As generally used, the term "input interface" refers to an apparatus which is configured for receiving at least one piece of data, specifically the data related to the reaction by the person as described above or below in more detail. For this purpose, the data can, preferably, be generated as input data by using a graphical user interface, and forwarded to the evaluation device for determining the desired data related to the at least one refractive value of an eye of the person. As generally used, the terms "graphical user interface" or "GUI" refer to a type of input interface which is configured to receive the desired data from a graphical interaction with a user. The graphical interaction may comprise presenting at least one of a menu or graphical icons, preferably including the visual stimulus, the virtual rotation element and the input element, on a screen to the user, and recording a reaction of the person by using the at least one input element. For this purpose, at least one touchscreen configured to provide access to inputting at least one piece of data may be used. In general, a clear, easy and intuitive user guidance may be preferred. As used herein, the term "guidance" refers to at least one piece of information provided to the person, preferably in a visual, acoustic or tactile fashion, which the at least one piece of information provides at least one of a recommendation to the person or a confirmation of an action performed by the person, specifically during a reaction of the person. However, using a graphical user interface having at least one further function may also be conceivable.

As further generally used, the term "output interface" refers to a further device which is configured to provide at least one output file comprising at least one further piece of data, specifically the desired data related to the at least one refractive value of an eye of the person. Herein, the processing device may, preferably, be configured to provide the data related to the at least one refractive value of an eye of the person the by using a different or, preferably, the same graphical user interface, which is configured for displaying the data comprised by the output file to the user. Alternatively or in addition, the output file may be transferred to a further processing device of the person, a further person, or entity, particularly selected from an optician, an ophthalmologist, or an insurance company, particularly for reviewing the data, and initiating a process for producing at least one spectacle lens or for approving the data and enabling the initiating of the process for producing the at least one spectacle lens However, further alternatives or using a further kind of output interface are conceivable.

According to a further aspect of the present invention, a computer program for determining at least one refractive value of an eye of a person is disclosed, comprising instructions which, when the program is executed by a computer, cause the computer to carry out the method for determining at least one refractive value of an eye of a person. As generally used, the term "computer program" refers to at least one executable instruction for at least one programmable device, specifically a computer, preferably a sequence of executable instructions, for processing and/or solving of at least one function and/or at least one task and/or at least one problem by using at least one programmable apparatus or device, specifically a computer, preferably for performing some or all steps of any one of the methods as described within the present invention. Typically, instructions are combined to a computer program code and/or provided in a programming language. A computer program is typically processed by using a processing device comprised by the at least one computer. For this purpose, the computer program may be running on the computer. The computer program code may be provided on a data storage medium or a separate device such as an optical storage medium, e.g., on a compact disc, directly on a computer or data processing device, or via a network, such as via an in-house network or via internet.

According to a further aspect, the present invention relates to a data carrier signal carrying outcome data which are generated by a method for determining at least one refractive value of an eye of a person which comprises generating the outcome data. As generally used, the term "data carrier signal" refers to an electronic signal comprising information. The data carrier signal may be provided on a computer-readable storage medium. As used herein, the term "computer-readable storage medium" specifically may refer to a non-transitory data storage means, such as a hardware storage medium having stored thereon computerexecutable instructions.

In a further aspect, the present invention relates to a method for producing at least one ophthalmic lens for an eye of a person, which comprises the following steps:
(i) determining at least one refractive value of an eye of a person by using the method according to any one of the preceding claims referring to a method for determining the at least one refractive value of the eye of the person;
(ii) generating at least one geometrical model of at least one ophthalmic lens by using the at least one refractive value of the eye of the person; and
(iii) processing at least one lens blank according to the at least one geometrical model of the at least one ophthalmic lens, whereby the at least one ophthalmic lens is produced.

For further details concerning the computer program, the data carrier signal, or the method for producing at least one ophthalmic lens, reference may be made to the method for determining at least one refractive value of an eye of a person as disclosed elsewhere herein.

In a further aspect, the present invention relates to an apparatus for determining at least one refractive value of an eye of a person. According to the present invention, the apparatus comprises:
- at least one projection device, wherein the at least one projection device is configured for displaying a visual stimulus to an eye of a person, wherein the visual stimulus comprises a plurality of concentric radial lines, wherein each radial line has a different angular value with respect to a center of the visual stimulus, wherein each radial line is projected at a particular defocus plane to the eye of the person, whereby a particular dioptric value is provided to the eye of the person;
- at least one input element, wherein the at least one input element is configured for recording a reaction by the person to the visual stimulus indicating that a particular radial line at a particular angular value has an appearance of maximum sharpness; and
- at least one evaluation device, wherein the at least one evaluation device is configured for determining at least one refractive value of the eye of the person by evaluating the reaction by the person to the visual stimulus.

In a particularly preferred embodiment, the apparatus may, further, comprise
- at least one rotation element, wherein the at least one rotation element is configured for rotating the visual stimulus by the person, wherein the angular value of each radial line with respect to the center of the visual stimulus is altered, whereby a rotated visual stimulus is obtained,
wherein the at least one input element is further configured for recording a reaction by the person to the rotated visual stimulus, wherein the at least one evaluation device is further configured for determining at least one refractive value of the eye of the person by evaluating the reaction by the person to the rotated visual stimulus.

In a particular embodiment, the apparatus may, further, comprise at least one distance meter. As used herein, the term "distance meter" refers to a device which is configured for determining at least one distance between an eye of the person and the at least one projection device configured for the displaying the at least one visual stimulus to an eye of the person. Herein, a value determined for the at least one distance between an eye of the person and the at least one projection device may be used in determining the at least one refractive value of the corresponding eye of the person. Preferably, the at least one distance meter is comprised by the desktop computer or the mobile communication device, particularly wherein at least one front camera of the mobile communication device configured for determining the at least one distance between an eye of the person and the at least one projection device may be used. However, further embodiments for the at least one distance meter may also be conceivable.

The apparatus can be provided in an arbitrary fashion as long as it is accessible to the person being desirous of determining at least one refractive value of an eye. In general, the apparatus can be provided in a room belonging to practice of an ophthalmologist or to a shop of an optician or optometrist. In a particular embodiment, the apparatus can be provided in a kiosk or a part thereof. As generally used, the term "kiosk" refers to an unmanned, free-standing structure which houses at least one of an interactive terminal or a video monitors, typically located in a public place, especially a shopping center or a retail store, being accessible by person for a use of the person. With regard to the present invention, the apparatus may be placed inside the kiosk and a person being desirous of determining at least one refractive value of an eye can approach the apparatus and perform the method as described elsewhere herein. In this manner, the at least one refractive value can be determined in a fashion which is applicable on a global scale to all kinds of persons, whereby difficulties with communication or compliance can be avoided as far as possible. However, providing the apparatus in a different fashion may also be conceivable.

For further details concerning the apparatus for determining at least one refractive value of an eye of a person, reference may be made to the method for determining at least one refractive value of an eye of a person as disclosed elsewhere herein.

The method, computer program, and apparatus for determining at least one refractive value of an eye of a person as disclosed herein, advantageously, allow determining at least one refractive value of an eye of a person, especially with regard to an astigmatic power and an astigmatic axis, by applying a reliable, easy and intuitive approach. In particular, the at least one refractive value can be determined without requiring an ophthalmologist, optician or optometrist, a set of measuring glasses or a sophisticated device, such as an autorefractive device, designated for this purpose. As indicated above, by using the method, computer program, and apparatus the at least one refractive value can be determined in a fashion which is applicable on a global scale to all kinds of persons over a broad range of cognitive skills, thereby avoiding difficulties with communication or compliance as far as possible.

As used herein, the terms "have", "comprise" or "include" or any arbitrary grammatical variations thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Summarizing, the following embodiments are particularly preferred within the scope of the present invention:
Embodiment 1. A method for determining at least one refractive value of an eye of a person, the method comprising the following steps:
   a) displaying a visual stimulus to an eye of a person, wherein the visual stimulus comprises a plurality of concentric radial lines, wherein each radial line has a different angular value with respect to a center of the visual stimulus, wherein each radial line is projected at a particular defocus plane to the eye of the person, whereby a particular dioptric value is provided to the eye of the person;
   b) recording a reaction by the person to the visual stimulus indicating that a particular radial line at a particular angular value has an appearance of maximum sharpness to the eye of the person; and
   c) determining at least one refractive value of the eye of the person by evaluating the reaction by the person to the visual stimulus.
Embodiment 2. The method according to the preceding embodiment, further comprising the following steps:
   d) rotating the visual stimulus by the person, wherein the angular value of each radial line with respect to the center of the visual stimulus is altered, whereby a rotated visual stimulus is obtained,
   wherein recording the reaction by the person to the visual stimulus according to step b) comprises indicating that a particular radial line at a particular angular value in the rotated visual stimulus has the appearance of maximum sharpness to the eye of the person, and wherein determining the at least one refractive value of the eye of the person according to step c) further comprises evaluating the reaction by the person to the rotated visual stimulus.
Embodiment 3. The method according to the preceding embodiment, wherein step d) comprises determining concurrently
   - a principal meridian from the particular angular value at which the particular radial line has the appearance of maximum sharpness to the eye of the person; and
   - a difference between the maximum value and the minimum value of the dioptric power from the particular dioptric value at which the particular radial line is projected at the particular defocus plane.
Embodiment 4. The method according to any one of the two preceding embodiments, further comprising the following steps:
   e) further rotating the visual stimulus by the person, whereby a further rotated visual stimulus is obtained, wherein the angular value of each radial line with respect to the center of the visual stimulus is further altered;
   f) recording a further reaction by the person to the further rotated visual stimulus indicating that a further particular radial line at a further particular angular value has the appearance of maximum sharpness to the eye of the person, wherein the further particular angular value is perpendicular to the particular angular value according to step d); and
   g) determining at least one further refractive value of the eye of the person by evaluating the further reaction by the person to the further rotated visual stimulus.
Embodiment 5. The method according to the preceding embodiment, wherein step g) comprises determining
   - a perpendicular meridian being perpendicular to the principal meridian from the further particular angular value at which the further particular radial line has the appearance of maximum sharpness to the eye of the person;
   - the maximum value of the dioptric power from a further particular dioptric value at which the further particular radial line is projected at the further particular defocus plane; and
   - the minimum value of the dioptric power by using the maximum value of the dioptric power and the difference between the maximum value and the minimum value of the dioptric power.
Embodiment 6. The method according to any one of the preceding embodiments, wherein displaying the visual stimulus to the eye of the person comprises using a projection device.
Embodiment 7. The method according to the preceding embodiment, wherein the projection device is configured for using a virtual display technique, wherein the projection device may, preferably, be selected from at least one of
   - a light field;
   - an holographic element;
   - a retinal laser beam projection.
Embodiment 8. The method according to any one of the preceding embodiments, wherein the radial lines are arranged as a fan having a starburst pattern around the center of the visual stimulus.
Embodiment 9. The method according to any one of the preceding embodiments, wherein visual stimulus is
   - a monochrome starburst pattern; or
   - a chromatic starburst pattern.
Embodiment 10. The method according to any one of the preceding embodiments, wherein the radial lines are arranged in equidistant steps.
Embodiment 11. The method according to any one of the preceding embodiments, wherein the visual stimulus comprises 6, 8, 9, 12, 15, 18, 24, 30, 36, 48, 60, 72, 90, 96, or 120 of radial lines.
Embodiment 12. The method according to any one of the preceding embodiments, wherein the defocus planes for adjacent radial lines are set by using a dioptric step.
Embodiment 13. The method according to the preceding embodiment, wherein the dioptric step is selected from a value of 0.125 D, 0.25 D, 0.5 D, 0.75 D, or 1.0 D.
Embodiment 14. The method according to any one of the preceding embodiments, wherein recording the reaction by the person comprises recording at least one of:
   - a tactile response by the person;
   - an acoustic response by the person;
   - a gesture of the person.
Embodiment 15. The method according to any one of the preceding embodiments, wherein the at least one refractive value of the eye of the person comprises at least one value of a refraction ellipse, in particular:
   - a first spatial direction indicating a maximum value or a minimum value of a dioptric power;
   - a second spatial direction indicating a minimum value of the dioptric power, if the first spatial direction indicates the maximum value of the dioptric power, or indicating a maximum value of the dioptric power, if the first spatial direction indicates the minimum value of the dioptric power;
   - an angular value indicating a direction of the maximum value or the minimum value of the dioptric power.
Embodiment 16. The method according to any one of the preceding embodiments, wherein the person is accompanied by a further person supporting the person, wherein the further person is, preferably, selected from at least one of a parent, a nurse, an optician, an apprentice, an ophthalmologist, a optics sales person, a technical photographer.
Embodiment 17. A computer program comprising instructions which, when the program is executed by a computer, cause the computer to carry out a method for determining at least one refractive value of an eye of a person according to any one of the preceding embodiments.
Embodiment 18. The computer program according to the preceding embodiment, wherein the computer program is stored on a computer-readable, non-transitory data carrier.
Embodiment 19. A data carrier signal carrying the outcome data generated by a method a method for determining at least one refractive value of an eye of a person according to any one of the preceding method embodiments.
Embodiment 20. A method for producing at least one ophthalmic lens for an eye of a person, the method comprising the following steps:
   (i) determining at least one refractive value of an eye of a person by using the method according to any one of the preceding embodiments referring to a method for determining the at least one refractive value of the eye of the person;
   (ii) generating at least one geometrical model of at least one ophthalmic lens by using the at least one refractive value of the eye of the person; and
   (iii) processing at least one lens blank according to the at least one geometrical model of the at least one ophthalmic lens, whereby the at least one ophthalmic lens is produced.
Embodiment 21. An apparatus for determining at least one refractive value of an eye of a person, the apparatus comprising:
   - at least one projection device, wherein the at least one projection device is configured for displaying a visual stimulus to an eye of a person, wherein the visual stimulus comprises a plurality of concentric radial lines, wherein each radial line has a different angular value with respect to a center of the visual stimulus, wherein each radial line is projected at a particular defocus plane to the eye of the person, whereby a particular dioptric value is provided to the eye of the person;
   - at least one input element, wherein the at least one input element is configured for recording a reaction by the person to the visual stimulus indicating that a particular radial line at a particular angular value has an appearance of maximum sharpness to the eye of the person; and
   - at least one evaluation device, wherein the at least one evaluation device is configured for determining at least one refractive value of the eye of the person by evaluating the reaction by the person to the visual stimulus.
Embodiment 22. The apparatus according to the preceding embodiment, further comprising:
   - at least one rotation element, wherein the at least one rotation element is configured for rotating the visual stimulus by the person, wherein the angular value of each radial line with respect to the center of the visual stimulus is altered, whereby a rotated visual stimulus is obtained,
   wherein the at least one input element is further configured for recording a reaction by the person to the rotated visual stimulus, wherein the at least one evaluation device is further configured for determining at least one refractive value of the eye of the person by evaluating the reaction by the person to the rotated visual stimulus.
Embodiment 23. The apparatus according to the preceding embodiment, wherein the at least one rotation element is further configured for further rotating the visual stimulus by the person, whereby a further rotated visual stimulus is obtained, wherein the angular value of each radial line with respect to the center of the visual stimulus is further altered; wherein the at least one input element is further configured for recording a further reaction by the person to the further rotated visual stimulus indicating that a further particular radial line at a further particular angular value has the appearance of maximum sharpness to the eye of the person, wherein the further particular angular value is perpendicular to the particular angular value according to step d); and wherein the at least one evaluation device is further configured for determining at least one further refractive value of the eye of the person by evaluating the further reaction by the person to the further rotated visual stimulus.
Embodiment 24. The apparatus according to any one of the preceding apparatus embodiments, wherein the at least one projection device is further configured to display a rotation of the at least one visual stimulus by the angular value.
Embodiment 25. The apparatus according to any one of the preceding apparatus embodiments, wherein the at least one projection device is selected from
   - a monitor comprised by a desktop computer; or
   - a touchscreen comprised by a mobile communication device.
Embodiment 26. The apparatus according to the preceding embodiment, wherein the mobile communication device is selected from at least one of:
   - a smartphone;
   - a smart watch;
   - a smart glass;
   - an augmented reality glass;
   - a virtual reality glass;
   - a laptop;
   - a tablet;
   - a smart television.
Embodiment 27. The apparatus according to any one of the preceding apparatus embodiments, further comprising
   - at least one holding element attached to the projection device, wherein the at least one holding element is configured to maintain a position of the projection device with respect to the eye of the person.
Embodiment 28. The apparatus according to any one of the preceding apparatus embodiments, wherein the at least one rotation element is selected from at least one of:
   - a mechanical rotation element;
   - a virtual rotation element.
Embodiment 29. The apparatus according to the preceding embodiment, wherein the mechanical rotation element is selected from at least one of a turning knob, a button, or a switch.
Embodiment 30. The apparatus according to any one of the two preceding embodiments, wherein the virtual rotation element is selected from at least one of a virtual turning knob, a virtual button, or a virtual switch.
Embodiment 31. The method according to the three preceding embodiments, wherein the virtual rotation element is displayed on a touchscreen.
Embodiment 32. The apparatus according to the preceding embodiment, wherein the touchscreen is comprised by the projection device configured for displaying the visual stimulus to the eye of the person.
Embodiment 33. The apparatus according to any one of the preceding apparatus embodiments, wherein the at least one input element is configured for recording at least one of
   - a tactile response by the person;
   - an acoustic response by the person;
   - a gesture of the person.
Embodiment 34. The apparatus according to the preceding embodiment, wherein the at least one tactile response by the person is recorded by using at least one of a mechanical button, or a virtual button.
Embodiment 35. The apparatus according to the preceding embodiment, wherein the virtual button is displayed on the touchscreen.
Embodiment 36. The apparatus according to any one of the three preceding embodiments, wherein the at least one acoustic response by the person is recorded by using a microphone.
Embodiment 37. The apparatus according to any one of the four preceding embodiments, wherein the at least one gesture of the person is recorded by using at least one of a camera, a motion sensor, or an eye tracker.
Embodiment 38. The apparatus according to any one of the preceding apparatus embodiments, further comprising
   - at least one distance meter, wherein the at least one distance meter is configured for determining at least one distance between the eye of the person and the at least one projection device configured for the displaying the at least one visual stimulus to the eye of the person.
Embodiment 39. The apparatus according to the preceding embodiment, wherein the at least one distance meter is comprised by the mobile communication device, particularly wherein at least one front camera of the mobile communication device is configured for determining the at least one distance between the eye of the person and the at least one projection device.
Embodiment 40. The apparatus according to any one of the preceding apparatus embodiments, wherein the at least one evaluation device is or is comprised by at least one of
   - a desktop computer
   - a mobile communication device
   - a server;
   - a cloud server.

### Short description of the Figures

Further optional features and embodiments of the present invention are disclosed in more detail in the subsequent description of preferred embodiments, preferably in conjunction with the dependent claims. Therein, the respective optional features may be realized in an isolated fashion as well as in any arbitrary feasible combination, as the skilled person will realize. It is emphasized here that the scope of the invention is not restricted by the preferred embodiments. In the Figures:
- Figure 1: illustrates a preferred embodiment of an apparatus for determining at least one refractive value of an eye of a person according to the present invention;
- Figures 2 to 5: each illustrates a visual stimulus in connection with a preferred method of determining the at least one refractive value; and
- Figure 6: illustrates a preferred embodiment of a method for producing a spectacle lens for an eye of the person according to the present invention.

### Detailed description of the embodiments

Figure 1 illustrates a preferred embodiment of an apparatus 110 for determining at least one refractive value of an eye 112 of a person 114. Herein, the at least one refractive value of both eyes 112 of the person 114 can, preferably, be determined in a consecutive fashion. The apparatus 110 can be provided in a room belonging to practice of an ophthalmologist or to a shop of an optician or optometrist. As an alternative, the apparatus 110 can be provided in a kiosk or a part thereof. In this manner, the person 114 has easy access to the apparatus 110 at a public place, especially a shopping center or a retail store, allowing determining the at least one refractive value on a global scale to all kinds of persons 114 without difficulties with communication or compliance as far as possible.

As schematically shown, the exemplary apparatus 110 of Figure 1 comprises - without limiting the scope of the invention - an electronic device 116 having a projection device 118 which is configured for displaying a visual stimulus image 120 to the user 114. As illustrated there, the electronic device 116 comprises a monitor 122 and a corresponding desktop computer 124. As an alternative (not depicted here), a touchscreen comprised by a mobile communication device can be used, wherein the mobile communication device may, preferably, be selected from at least one of a smartphone, a smart watch, a smart glass, an augmented reality glass, a virtual reality glass, a laptop, a tablet, or a smart television. However, further alternatives may also be feasible.

Figures 1 to 5 schematically illustrated the exemplary visual stimulus 120 having a plurality of concentric radial lines 126, 126', ..., wherein each radial line 126, 126', ... has a different angular value 128, 128', ... with respect to a center 130 of the visual stimulus 120. In the particularly preferred exemplary embodiment as shown in Figures 1 to 5, the concentric radial lines 126, 126', ... are arranged as a fan having a starburst pattern around the center 130 of the visual stimulus in a sense as disclosed by Keirl A.W. et al., see above, in connection with the so-denoted "fan-and-block-technique. In this manner, a so-denoted "starburst pattern" is provided, which appears to originate from the center 130 of the visual stimulus 120. As depicted there, the visual stimulus 120 is a monochrome starburst pattern; however, a chromatic starburst pattern may also be feasible.

Although each radial line 126, 126', ... directs to the center 130 of the visual stimulus 120, it originates in a particular distance from the center 130 of the visual stimulus 120. As further illustrated in Figures 1 to 5, concentric radial lines 126, 126', ... are arranged in equidistant steps; however, using different angular distances between adjacent radial lines 126, 126', ... may also be feasible. The exemplary visual stimulus 120 as depicted in Figures 1 to 5 comprises 18 concentric radial lines radial lines 126, 126', ..., which results in an angular distance of 20° between adjacent radial lines 126, 126', ...; however, using a different number of concentric radial lines 126, 126', ... may also be feasible. Still, various further embodiments of the visual stimulus 120 are conceivable.

In accordance with the present invention, each radial line 126, 126', ... is projected at a particular defocus plane to the eye 112 of the person 114, thereby providing a particular dioptric value to the eye 112 of the person 114. Although the eye 112 of the person 114 is located at a constant distance 132 from the projection device 118, it recognizes each radial line 126, 126', ... as having a particular distance from the eye 112 of the person 114, wherein the particular distance from the eye 112 of the person 114 may, preferably, be different for each radial line 126, 126', .... As further depicted in Figures 2 to 5, a dioptric step of 0,75 D is used as a difference between the defocus planes for adjacent radial lines radial lines 126, 126', ...; however, using a different value for the dioptric step may also be feasible. For a purpose of projecting each radial line 126, 126', ... at the particular defocus plane with respect to the eye 112 of the person 114, the projection device 118 may, preferably, be configured for using a virtual display technique, particularly be selected from a light-field, a holographic optical element, and/or a retinal laser beam projection; however, using a different type of projection device may also be conceivable.

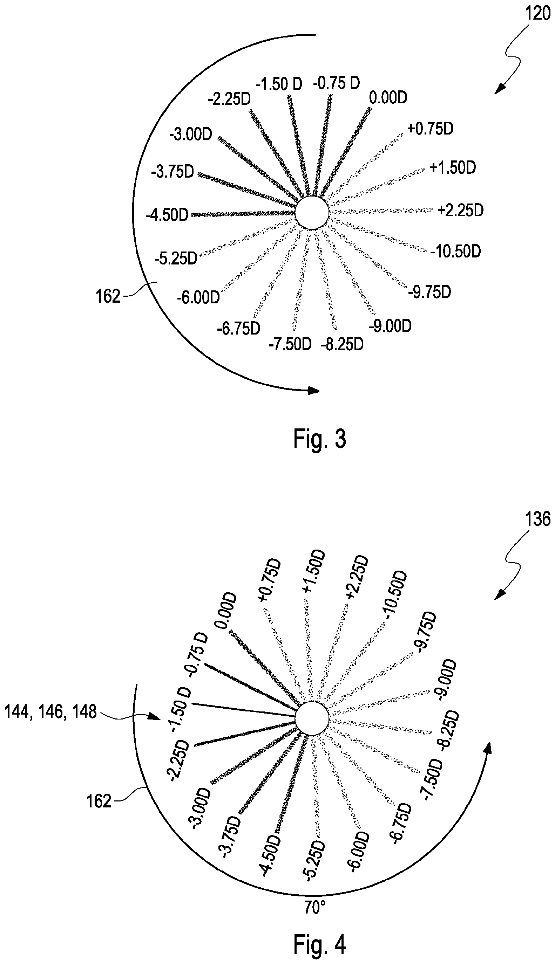

While each radial line 126, 126', ... of the visual stimulus 120 as presented on the projection device 118 to the eye 112 of the person 114 according to Figures 1 and 2, is a continuous elongated line having a uniform lateral extension and an identical sharpness, the eye 112 of the person 114 recognizes a difference in sharpness, depending on the at least one refractive value of the eye 112 of the person 114 and a relative orientation of the visual stimulus 120 with respect to the eye 112 of the person 114, as schematically depicted in Figures 3 to 5.

For a purpose of altering the relative orientation of the visual stimulus 120 with respect to the eye 112 of the person 114, the apparatus 110 further comprises a rotation element 134. The rotation element 134 is configured for rotating the visual stimulus 120 by the person 114 in a manner that the angular value of each concentric radial line 126, 126', ... with respect to the center 130 of the visual stimulus is altered, whereby a rotated visual stimulus 136 is obtained. As schematically illustrated in Figure 1, the rotation element 134 is a turning knob 138, which can be touched by the hand 140 of the person 114 in order to induce a rotation of the visual stimulus 120 as displayed on the projection device 118. Herein, an intensity, a duration and/or a direction of the touching of the rotation element 134 by the hand 140 of the person 114 induces a velocity and/or a direction of the rotation of the visual stimulus 120. However, using a different kind of mechanical rotation element, in particular a button, or a switch, may also be feasible. Preferably, the mechanical rotation element may be placed in a vicinity of the projection device 118 in order to facilitate the concentration of the person 114 towards the visual stimulus 120 while the person 114 is using the rotation element.

Alternatively or in addition (not depicted here), a virtual rotation element may be displayed on the projection device 118 close to the visual stimulus 120, but not overlapping it. In particular, the virtual rotation element may be a virtual turning knob, a virtual button, or a virtual switch, resembling the mechanical equivalent to the person 134; however, using a different kind of virtual element may also be feasible. As a further alternative or still in addition (not depicted here), the virtual rotation element may be configured for voice control by the person 114, especially by recording an acoustic response by the person 114. For this purpose, the apparatus 110 may comprise a microphone 142 as schematically depicted in Figure 1. However, still further embodiments of the virtual rotation element are conceivable.

For a purpose of indicating that a particular radial line 144 at a particular angular value 146 has an appearance of maximum sharpness 148 to the eye 112 of the person 114, the apparatus 110 further comprises an input element 150. The input element 150 is configured for recording a reaction by the person 114 to the visual stimulus 120 or, preferably, to the rotated visual stimulus 136 indicating that the particular radial line 144 at the particular angular value 146 appears to the eye 112 of the person 114 at maximum sharpness 148 as schematically illustrated in Figure 4. In the exemplary embodiment of Figure 1, the input element 150 and the rotation element 134 constitute a combined rotation and input element 152, which is configured here for providing functions of both the rotation element 134 and the input element 150. In this particular embodiment, the turning knob 138 can further be pressed by the hand 140 of the person 114 in order to indicate the desired reaction by the person 114 to the rotated visual stimulus 136 expressing that the particular radial line 144 at the particular angular value 146 appears at maximum sharpness 148 to the eye 112 of the person 114. As an alternative, removing the hand 140 of the person 114 from the turning knob 138 may, initially, induce a stop of the rotation of the visual stimulus 120 and, after a predefined time interval, be interpreted as the desired reaction by the person 114 to the visual stimulus 120 or, preferably, to the rotated visual stimulus 136. However, using a different type of input element 150 may also be feasible.

Apart from recording a tactile response by the hand 140 of the person 114 by using the input element 150 as depicted in Figure 1, an acoustic response by the person 114 and/or a gesture of the person 140 can be used for recording the desired reaction by the person 114 to the visual stimulus 120 or, preferably, to the rotated visual stimulus 136. For a purpose of recording the acoustic response by the person 114, the microphone 142 as comprised by the exemplary apparatus 110 of Figure 1 can, further, be used. For a purpose of recording the at least one gesture of the person, the apparatus 110 may comprise a monitoring device, in particular a front camera 154 as schematically depicted in Figure 1; however, using a different arrangement and type of the monitoring device may also be conceivable. Herein, the front camera 154 may be used as a camera, a motion sensor, or an eye tracker. Further, the front camera 154 may be used as a distance meter for determining the distance 132 between the eye 112 of the person 114 and the projection device 118.

For a purpose of determining at least one refractive value of the eye 112 of the person 114, the apparatus 110 further comprises an evaluation device 156. The evaluation device 156 is configured for determining the at least one refractive value of the eye 112 of the person 114 by evaluating the reaction by the person 114 to the visual stimulus 120 or, preferably, to the rotated visual stimulus 136. As shown in Figure 1, the evaluation device 156 may, preferably be comprised by the desktop computer 124. However, fully or partially using a further local processing device and/or a remote processing device (not depicted here), in particular a server or a cloud server, may also be feasible.

In addition, a graphical user interface 158 configured for assisting the person 114 at determining the at least one refractive value of the eye 112 of the person 114 may be displayed on the projection device 118. In particular, the graphical user interface 158 may show at least one piece of information about the person 114, the at least one refractive value of the eye 112 of the person 114 and/or one or more steps of related procedure for determining the at least one refractive value of the eye 112 of the person 114. By way of example, at least one piece of information about rotating the visual stimulus 120 and/or the reaction by the person 114 to the rotated visual stimulus 136 can be provided to the person 114 or initiated by providing the at least one corresponding piece of information to the person 114. As further depicted in Figure 1, the apparatus 110 further comprises a loudspeaker 160, which may be configured to support the graphical user interface 158 by providing the at least one piece of information to the person 114 in an acoustic fashion.

Figures 2 illustrates an enlarged version of the same visual stimulus 120 as displayed on the projection device 118 as shown in Figure 1. As depicted here, the visual stimulus 120 comprises 18 concentric radial lines radial lines 126, 126', ... in an angular distance of 20° between adjacent radial lines 126, 126', ..., which have been projected by using a virtual display technique, whereby 18 different defocus planes ranging from +2.25 D to -10.50 D in 0.75 D dioptric steps are used. As further indicated by the arrow 162, the visual stimulus 120 can be rotated by the person 14 by using the rotation element 134.

For determining the at least one refractive value of the eye 112 of the person 114, the visual stimulus 120 is used according to the method as disclosed herein to directly determine at least one value, preferably all values, of a refraction ellipse 164 configured to describe the refractive value s of the eye 112 of the person 114, wherein the values of the refraction ellipse 164 as further depicted there comprise:
- a first spatial direction D1 indicating a maximum value of a dioptric power, corresponding to a major axis of the refraction ellipse 164;
- a second spatial direction D2 indicating a minimum value of the dioptric power, corresponding to a minor axis of the refraction ellipse 164; and
- an angular value α with regard to the first spatial direction D1.

Accordingly, the starburst pattern as comprised by the visual stimulus 120, wherein each concentric radial line 126, 126', ... represents an individual refractive requirement for the eye 112 of the person 114, is rotated to the particular angular value 146 to appear under maximum sharpness 148 to the eye 112 of the person 114. By using continuous, elongated, concentric radial lines 126, 126', ..., for the visual stimulus 120, the contrast within the visual stimulus 120 may, predominantly, be a function of a broadening of a width of each line 126, 126', ..., which can be minimized by rotating the visual stimulus 120 in a manner that the rotated visual stimulus 136 is may be oriented parallel to the angular value α with regard to the first spatial direction D1 in order to compensate the maximum value of the dioptric power or, alternatively, parallel to an angular value 90° + α with regard to the first spatial direction D1 in order to compensate the minimum value of the dioptric power. As a result thereof, the orientation of the refraction ellipse 164 and one of its meridians can be measured by rotating the visual stimulus 120 alone without being visually affected by the astigmatism of the eye 112 of the person 114.

Thereafter, the visual stimulus 120 may be, further, rotated to maximize the contrast for a further particular radial line 166 at a further particular angular value 168 to appear under maximum sharpness 148 to the eye 112 of the person 114, wherein the further particular angular value 168 in the further rotated visual stimulus 170 is perpendicular to the particular angular value 146 as previously determined. As a result thereof, both the major axis and the minor axis of the refraction ellipse 164 configured to describe the refractive values of the eye 112 of the person 114 can been determined.

Figure 3 illustrates an example of a recognition of the visual stimulus 120 by the eye 112 of the person 114. Herein, the corresponding eye 112 of the person 114 has the following refractive values: Sph = -1.5 D, Cyl = -2.25 D, and A = 170°. As a result thereof, the first principal meridian has a dioptric power of -3.75 D at A = 80°, while the second principal meridian has a dioptric power of -1,5 D at A = 170°. As a consequence, the corresponding eye 112 of the person 114 recognizes the lines closer to the circle of least confusion of the person 114 less blurry compared to other lines; however, no line can be recognized with maximum sharpness 148 by the eye 112 of the person 114 under a rotation angle of the starburst pattern of the visual stimulus 120 as depicted in Figure 3.

The person 114 now rotates the starburst pattern as comprised by the visual stimulus 120 until the rotated visual stimulus 136 is obtained, wherein the particular line 144 at the particular angular value 146 appears at maximum sharpness 148 to the eye 112 of the person 114 as schematically illustrated in Figure 4. Moreover, the dioptric power of -1.5 D and its rotational position at A = 170° correspond to the respective values of the first principal meridian of the refraction ellipse 164 configured to describe the refractive values of the eye 112 of the person 114.

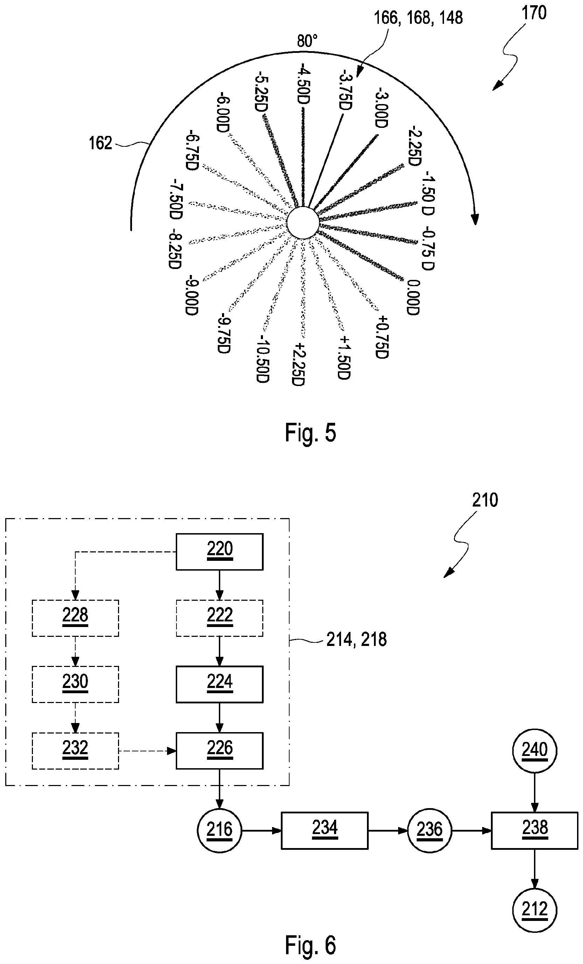

As illustrated in Figure 5, the person 114 further rotates the starburst pattern of the already rotated visual stimulus 136 until the further rotated visual stimulus 170 is obtained, wherein the further particular radial line 166 at the further particular angular value 168 appears under maximum sharpness 148 to the eye 112 of the person 114. As already indicated above, the further particular angular value 168 in the further rotated visual stimulus 170 according to Figure 5 is perpendicular to the particular angular value 146 as illustrated in Figure 4, showing sum -4.5 D of the dioptric powers of -1.5 D and -3.75D at the orientation of the further principal meridian at the axis of A = 80°. As a result thereof, the major axis and the minor axis of the refraction ellipse 164 configured to describe the refractive values of the eye 112 of the person 114 can be determined in this manner.

Figure 6 schematically illustrates a preferred embodiment of a method 210 for producing an ophthalmic lens 212 for the eye 112 of the person 114 according to the present invention.

In a refraction determining step 214 pursuant to method step (i), at least one refractive value 216 of the eye 112 of the person 114 is determined by using a method 218 for determining the at least one refractive value 216 of the eye 112 of the person 114.

Herein, the method 218 comprises a displaying step 220 pursuant to step a), according to which the visual stimulus 120 is displayed to the eye 112 of the person 114. As described above, the visual stimulus 120 comprises the plurality of the concentric radial lines 126, 126', ..., wherein each radial line 126, 126', ... has the different angular value 128, 128', ... with respect to the center 130 of the visual stimulus 120.

The method 218 may, further, comprise an optional rotating step 222 pursuant to step d), according to which the visual stimulus 120 may be rotated by the person 114, wherein the angular value 128, 128', ... of each radial line 126, 126', ... with respect to the center 130 of the visual stimulus 120 may be altered, whereby the rotated visual stimulus 136 may be obtained, such as exemplarily depicted in Figure 4.

Further, the method 218 for determining the at least one refractive value 216 of the eye 112 of the person 114 comprises a recording step 224 pursuant to step b), according to which the reaction by the person 114 to the visual stimulus 120 or, preferably, to the rotated visual stimulus 136 is recorded.

Further, the method 218 comprises a determining step 226 pursuant to step c), according to which the at least one refractive value 216 of the eye 112 of the person 114 is determined by evaluating the reaction by the person 114 to the visual stimulus 120 or, preferably, to the rotated visual stimulus 136.

In a particularly preferred embodiment, the method 218 may further comprise a further rotating step 228 pursuant to step e), according to which the visual stimulus 120 is further rotated by the person 114, such as exemplarily depicted in Figure 5, whereby the further rotated visual stimulus 170 is obtained, wherein the angular value 128, 218', ... of each radial line 126, 126', ... with respect to the center 130 of the visual stimulus 120 is further altered.

In this particularly preferred embodiment, the method 218 may further comprise a further recording step 230 pursuant to step f), according to which a further reaction by the person 114 to the further rotated visual stimulus 170 is recorded. Herein, further reaction by the person 114 to the further rotated visual stimulus 170 indicates that a further particular radial line 166 at a further particular angular value 168 has the appearance of maximum sharpness 148 to the eye 112 of the person 114. The further particular angular value 168 is perpendicular to the particular angular value 146 as recorded according to the recording step 224 pursuant to step d).

In this particularly preferred embodiment, the method 218 may further comprise a further determining step 232 pursuant to step g), according to which at least one further refractive value 216 of the eye 112 of the person 114 is determined by evaluating the further reaction by the person 114 to the further rotated visual stimulus 170. Herein, the at least one further value as determined here by applying the further determining step 232 is attributed to the at least one refractive value 216 of the eye 112 of the person 114 as determined above in the determining step 226 pursuant to step d).

Further, the method 210 for producing the at least one ophthalmic lens 212 for the eye 112 of the person 114 comprises a generating step 234 according to method step (ii), wherein at least one geometrical model 236 of the at least one ophthalmic lens 212 is generated by using the at least one refractive value 216 of the eye 112 of the person 114 as determined by using the refraction determining step 214 pursuant to method step (i).

Further, the method 210 for producing the at least one ophthalmic lens 212 for the eye 112 of the person 114 comprises a processing step 238 according to method step (iii), wherein at least one lens blank 240 is processed according to the at least one geometrical model 236 of the at least one ophthalmic lens 212, whereby the at least one ophthalmic lens 212 is produced.

### List of Reference Signs

- 110: apparatus for determining at least one refractive value of an eye of a person
- 112: eye
- 114: person
- 116: electronic device
- 118: projection device
- 120: visual stimulus
- 122: desktop computer
- 124: desktop computer
- 126, 126', ...: concentric radial line
- 128, 128', ...: angular value
- 130: center
- 132: distance
- 134: rotation element
- 136: rotated visual stimulus
- 138: turning knob
- 140: hand
- 142: microphone
- 144: particular radial line
- 146: particular angular value
- 148: maximum sharpness
- 150: input element
- 152: combined rotation and input element
- 154: front camera
- 156: evaluation device
- 158: graphical user interface
- 160: loudspeaker
- 162: arrow
- 164: refraction ellipse
- 166: further particular radial line
- 168: further particular angular value
- 170: further rotated visual stimulus
- 210: method for producing the at least one ophthalmic lens
- 212: ophthalmic lens
- 214: refraction determining step
- 216: at least one refractive value of the eye of the person
- 218: method for determining at least one refractive value of the eye of the person
- 220: displaying step
- 222: rotating step
- 224: recording step
- 226: determining step
- 228: further rotating step
- 230: further recording step
- 232: further determining step
- 234: generating step
- 236: geometrical model of the at least one ophthalmic lens
- 238: processing step
- 240: lens blank

## Claims

1. A method (218) for determining at least one refractive value (216) of an eye (112) of a person (114), the method (218) comprising the following steps:
a) (220) displaying a visual stimulus (120) to an eye (112) of a person (114), wherein the visual stimulus (120) comprises a plurality of concentric radial lines (126, 126,', ...), wherein each radial line (126, 126,', ...) has a different angular value (128, 128,', ...) with respect to a center (130) of the visual stimulus (120);
b) (224) recording a reaction by the person (114) to the visual stimulus (120); and
c) (226) determining at least one refractive value (216) of the eye (112) of the person (114) by evaluating the reaction by the person (114) to the visual stimulus (120),
**characterized in**
**that** step a) (220) comprises that each radial line (126, 126,', ...) is projected at a particular defocus plane to the eye (112) of the person (114), whereby a particular dioptric power is provided to the eye (112) of the person (114), and that step b) (224) comprises that recording the reaction by the person (114) to the visual stimulus (120) indicates that a particular radial line (144) at a particular angular value (146) has an appearance of maximum sharpness (148) to the eye (112) of the person (114).

2. The method (218) according to the preceding claim, further comprising the following step:
d) (222) rotating the visual stimulus (120) by the person (114), wherein the angular value (128, 128,', ...) of each radial line (126, 126,', ...) with respect to the center (130) of the visual stimulus (120) is altered, whereby a rotated visual stimulus (136) is obtained,
wherein recording the reaction by the person (114) to the visual stimulus (120) according to step b) comprises indicating that a particular radial line (144) at a particular angular value (146) in the rotated visual stimulus (136) has the appearance of maximum sharpness (148) to the eye (112) of the person (114), and wherein determining the at least one refractive value (216) of the eye (112) of the person (114) according to step c) further comprises evaluating the reaction by the person (114) to the rotated visual stimulus (136).

3. The method (218) according to the preceding claim, wherein step d) (226) comprises determining concurrently
- a principal meridian from the particular angular value at which the particular radial line (144) has the appearance of maximum sharpness (148) to the eye (112) of the person (114); and
- a difference between the maximum value and the minimum value of the dioptric power from the particular dioptric value at which the particular radial line (144) is projected at the particular defocus plane.

4. The method (218) according to any one of the two preceding claims, further comprising the following steps:
e) (228) further rotating the visual stimulus (120) by the person (114), whereby a further rotated visual stimulus (170) is obtained, wherein the angular value (128, 128,', ...) of each radial line (126, 126,', ...) with respect to the center (130) of the visual stimulus (120) is further altered;
f) (230) recording a further reaction by the person (114) to the further rotated visual stimulus (170) indicating that a further particular radial line (166) at a further particular angular value (168) has the appearance of maximum sharpness (148) to the eye (112) of the person (114), wherein the further particular angular value (168) is perpendicular to the particular angular value (146) according to step d) (224); and
g) (232) determining at least one further refractive value (216) of the eye (112) of the person (114) by evaluating the further reaction by the person (114) to the further rotated visual stimulus (170).

5. The method (218) according to the preceding claim, wherein step g) (232) comprises determining
- a perpendicular meridian being perpendicular to the principal meridian from the further particular angular value (168) at which the further particular radial line (166) has the appearance of maximum sharpness (148) to the eye (112) of the person (114);
- the maximum value of the dioptric power from a further particular dioptric value at which the further particular radial line (166) is projected at the further particular defocus plane; and
- the minimum value of the dioptric power by using the maximum value of the dioptric power and the difference between the maximum value and the minimum value of the dioptric power.

6. The method (218) according to any one of the preceding claims, wherein displaying the visual stimulus (120) to the eye (112) of the person (114) comprises using a projection device (118), wherein the projection device (118) is configured for using a virtual display technique, wherein the projection device (118) is, particularly, selected from at least one of a light field, an holographic element, or a retinal laser beam projection.

7. The method (218) according to any one of the preceding claims, wherein the radial lines (126, 126,', ...) are arranged as a fan having a starburst pattern around the center (130) of the visual stimulus (120).

8. The method (218) according to any one of the preceding claims, wherein the radial lines (126, 126,', ...) are arranged in equidistant steps.

9. The method (218) according to any one of the preceding claims, wherein the defocus planes for adjacent radial lines (126, 126,', ...) are set by using a dioptric step.

10. The method (218) according to any one of the preceding claims, wherein recording the reaction by the person (114) comprises recording at least one of:
- a tactile response by the person (114);
- an acoustic response by the person (114);
- a gesture of the person (114).

11. A computer program comprising instructions which, when the program is executed by a computer, cause the computer to carry out a method (218) for determining at least one refractive value (216) of an eye (112) of a person (114), the method (218) comprising the following steps:
a) (220) displaying a visual stimulus (120) to an eye (112) of a person (114), wherein the visual stimulus (120) comprises a plurality of concentric radial lines (126, 126,', ...), wherein each radial line (126, 126,', ...) has a different angular value (128, 128,', ...) with respect to a center (130) of the visual stimulus (120);
b) (224) recording a reaction by the person (114) to the visual stimulus (120); and
c) (226) determining at least one refractive value (216) of the eye (112) of the person (114) by evaluating the reaction by the person (114) to the rotated visual stimulus (136),
**characterized in**
**that** step a) (220) comprises that each radial line (126, 126,', ...) is projected at a particular defocus plane to the eye (112) of the person (114), whereby a particular dioptric power is provided to the eye (112) of the person (114), and that step b) (224) comprises that recording the reaction by the person (114) to the visual stimulus (120) indicates that a particular radial line (144) at a particular angular value (146) has an appearance of maximum sharpness (148) to the eye (112) of the person (114).

12. A method (210) for producing at least one ophthalmic lens (212) for an eye (112) of a person (114), the method (210) comprising the following steps:
(i) (214) determining at least one refractive value (216) of an eye (112) of a person (114) by using the method (218) according to any one of the preceding claims referring to a method (218) for determining the at least one refractive value (216) of the eye (112) of the person (114);
(ii) (234) generating at least one geometrical model (236) of at least one ophthalmic lens (212) by using the at least one refractive value (216) of the eye (112) of the person (114); and
(iii) (238) processing at least one lens blank (240) according to the at least one geometrical model (236) of the at least one ophthalmic lens (212), whereby the at least one ophthalmic lens (212) is produced.

13. An apparatus (110) for determining at least one refractive value (216) of an eye (112) of a person (114), the apparatus (110) comprising:
- at least one projection device (118), wherein the at least one projection device (118) is configured for displaying a visual stimulus (120) to an eye (112) of a person (114), wherein the visual stimulus (120) comprises a plurality of concentric radial lines (126, 126,', ...), wherein each radial line (126, 126,', ...) has a different angular value (128, 128,', ...) with respect to a center (130) of the visual stimulus (120);
- at least one input element (150), wherein the at least one input element (150) is configured for recording a reaction by the person (114) to the visual stimulus (120); and
- at least one evaluation device (156), wherein the at least one evaluation device (156) is configured for determining at least one refractive value (216) of the eye (112) of the person (114) by evaluating the reaction by the person (114) to the visual stimulus (120),
**characterized in**
- **that** the at least one projection device (118) is further configured for displaying the visual stimulus (120) to the eye (112) of the (114) person that each radial line (126, 126,', ...) is projected at a particular defocus plane to the eye (112) of the person (114), whereby a particular dioptric value is provided to the eye (112) of the person (114), and
- **that** the at least one input element (150) is further configured for recording the reaction by the person (114) to the visual stimulus (120) indicating that a particular radial line (144) at a particular angular value (146) has an appearance of maximum sharpness (148) to the eye (112) of the person (114).

14. The apparatus (110) according to the preceding claim, further comprising
- at least one rotation element (134), wherein the at least one rotation element (134) is configured for rotating the visual stimulus (120) by the person (114), wherein the angular value (128, 128,', ...) of each radial line (126, 126,', ...) with respect to the center (130) of the visual stimulus (120) is altered, whereby a rotated visual stimulus (136) is obtained,
wherein the at least one input element (150) is further configured for recording a reaction by the person (114) to the rotated visual stimulus (136), wherein the at least one evaluation device (156) is further configured for determining at least one refractive value (216) of the eye (112) of the person (114) by evaluating the reaction by the person (114) to the rotated visual stimulus (136).

15. The apparatus (110) according to the preceding claim, wherein the at least one rotation element (134) is further configured for further rotating the visual stimulus (120) by the person (114), whereby a further rotated visual stimulus (170) is obtained, wherein the angular value (128, 128,', ...) of each radial line (126, 126,', ...) with respect to the center (130) of the visual stimulus (120) is further altered; wherein the at least one input element (150) is further configured for recording a further reaction by the person (114) to the further rotated visual stimulus (170) indicating that a further particular radial line (166) at a further particular angular value (168) has the appearance of maximum sharpness (144) to the eye (112) of the person (114), wherein the further particular angular value (168) is perpendicular to the particular angular value (146) according to step d); and wherein the at least one evaluation device (156) is further configured for determining at least one further refractive value (216) of the eye (112) of the person (114) by evaluating the further reaction by the person (114) to the further rotated visual stimulus (170).
